# EUROPÄISCHE PATENTSCHRIFT

(11) **EP 1 530 982 B1**
(45) Veröffentlichungstag und Bekanntmachung des Hinweises auf die Patenterteilung: **23.07.2008**
(21) Anmeldenummer: 04026064.8
(22) Anmeldetag: 03.11.2004
(51) Int. Cl.: A61N 1/32, H03K 17/78

(54) **Vorrichtung zur Elektrotherapie**
Electrotherapy device
Dispositif d'électrothérapie

(30) Priorität: 13.11.2003 DE 10353000
(43) Veröffentlichungstag der Anmeldung: 18.05.2005
(73) Patentinhaber: Physiomed Elektromedizin AG, 91220 Schnaittach/Laipersdorf (DE)
(72) Erfinder: Reinhold, Walter, 91220 Schnaittach (DE)
(74) Vertreter: Schneck, Herbert

(56) Entgegenhaltungen:
- DE-A- 4 040 164
- DE-U- 8 630 690
- GB-A- 1 444 985
- US-A- 3 671 749
- US-A- 4 976 263
- US-A- 5 002 034

## Beschreibung

Die Erfindung richtet sich auf eine Vorrichtung zur Elektrotherapie, insbesondere mit einer Schaltungsanordnung mit einer Endstufe zur Erzeugung einer Folge von Spannungsimpulsen und mit zwei Elektroden zum Anlegen dieser Spannungsimpulse an ein zu behandelndes Körperteil, wobei eine der beiden Elektroden mit einer Oberfläche mit einem relativ hohen Übergangswiderstand und die andere Elektrode mit einem relativ geringen Übergangswiderstand zur menschlichen Haut ausgebildet ist, wobei die Elektrode mit der Oberfläche mit dem hohen Übergangswiderstand über das zu behandelnde Körperteil bewegbar ist und die Oberfläche durch eine Kunststoffschicht gebildet ist, wobei Impulsfolgen mit Spannungen von 1 bis 600 V erzeugt werden und im Gewebe einen Strom im Mikroampere-Bereich hervorgerufen wird. Eine Vorrichtung gemäß Oberbegriff des Anspruchs 1 ist bekannt aus US-A 4976263 oder DE 37 16 816 C2.

Durch eine derartige Vorrichtung wird erreicht, dass in dem menschlichen Gewebe selbst nur Ströme äußerst geringer Stromstärke, d.h. im Mikroamperebereich, fließen. Dies ist durch den hohen Übergangswiderstand der wenigstens einen Elektrode bedingt, wobei durch eine Strombegrenzungseinrichtung sichergestellt ist, dass auch bei vom Regelfall abweichenden Gegebenheiten, z.B. bei einem hohen Feuchtigkeitsgehalt der Umgebung, keine Gefahr für die zu behandelnde Person aufgrund der verwendeten relativ hohen Spannung besteht.

Durch die an das zu behandelnde Körperteil angelegten Impulsfolgen wird die Massagewirkung auf das Gewebe periodisch entsprechend der erregenden Frequenz moduliert, wodurch eine äußerst wirksame Entstauung von Lymphflüssigkeit, von übersäuerter Muskulatur, Blutergüssen oder dergleichen durch eine autonome Regeneration des Bindegewebes erzielt werden kann, d.h. die Konzentrationsanhäufung im Gewebe wird durchbrochen.

Die Wirksamkeit derartiger Massageeinrichtungen ist durch eine endogene Modulation bedingt. Für die behandelte Person ebenso wie für den Therapeuten ist lediglich ein körpereigenes Pulsieren bzw. Vibrieren wahrnehmbar. Darüber hinaus können bei dieser Art der Erregung Frequenzen eingesetzt werden, welche mittels einer rein mechanischen Anregung nur schwer realisierbar sind. Durch Anregung entsprechender Impulsmuster ist die genaue Steuerung der Dynamik der Vibrationsbewegung möglich.

Der für die Wirksamkeit einer derartigen Vorrichtung verantwortliche Effekt beruht unter anderem auf einer periodischen Änderung der Reibungskraft zwischen dem Handschuh des Therapeuten bzw. der vom Therapeuten bewegten Elektrode und dem behandelten Gewebe aufgrund einer dem Johnson-Raabeck-Effekt ähnlichen Erscheinung. Entscheidend für das Auftreten dieses Effekts ist ein halbleiterartiges Verhalten einer der Elektroden, nämlich der bewegten Elektrode.

Das pulsierende elektrische Feld zwischen der Hand des Therapeuten und dem Körper des Patienten führt zu einer pulsierenden elektrostatischen Anziehungskraft und damit zu einer pulsierenden Reibungskraft und diese wiederum letztlich zu einer pulsierenden Verformung des Gewebes, welche auch fühlbar ist.

Hiervon ausgehend liegt der Erfindung die Aufgabe zugrunde, die an sich bekannte Wirkungsweise einer derartigen Vorrichtung, die sich außerordentlich bewährt hat, noch weiter zu steigern.

Dementsprechend ist zur Lösung dieser Aufgabe vorgesehen, dass eine Schnellentladeschaltung (E) mehrere durch Optokoppler angesteuerte Thyristoren (N1 bis N4) umfasst, wobei einer Grundfrequenz in der Größenordnung von 100 Hz eine Frequenz in der Größenordnung von 1 bis 10 Hz überlagert ist.

Es ist an sich bekannt, bei derartigen Vorrichtungen Schnellentladeschaltungen vorzusehen, die wegen der fehlenden Selbstentladung der Elektrode mit dem hohen Übergangswiderstand erforderlich sind. Erfindungsgemäß wird eine derartige Selbstentladeschaltung so ausgestaltet, dass sie mehrere durch Optokoppler angesteuerte Thyristoren umfasst. Es ist auf diese Weise möglich, positive und negative Impulse im Wechsel zu erzeugen, welche der sich einstellenden Polarisierung der Folie entgegenwirken, insbesondere kann hierdurch aber auch eine höhere Spannungsfrequenz erzeugt werden, was die Wirksamkeit der Behandlung deutlich erhöht.

Bei Reizstromgeräten lässt sich eine solche Schaltung vorteilhaft zum Generieren von biphasischen Impulsen einsetzen, welche herkömmlicherweise mit Hilfe von Relais erzeugt werden.

Es ist zwar an sich schon bekannt, bei derartigen Vorrichtungen eine Frequenzmodulation der Impulse vorzunehmen, wobei bei der gattungsgemäß als bekannt vorausgesetzten Vorrichtung von einer Grundfrequenz von 30 Hz ausgegangen wurde.

Demgegenüber wurde erfindungsgemäß nun gefunden, dass eine besonders hohe Wirksamkeit dadurch erreicht werden kann, dass die einander überlagerten Frequenzen sich relativ stark, z.B. größenordnungsmäßig um einen Faktor 100, unterscheiden. Es kann also einerseits mit einer relativ hohen Frequenz von beispielsweise 120 Hz und andererseits mit einer relativ niedrigen Frequenz von 1 Hz gearbeitet werden.

Die höhere Grundfrequenz entsprechend der Resonanzschwingung der Lymphflüssigkeit bewirkt durch ein Aufwühlen von lymphpflichtiger Last, also z.B. von Eiweißen, dass diese transportfähig werden bzw. wieder transportfähig gemacht werden, während die niedrigere Frequenz, als sogenannte "Burst-Frequenz", die Lymph-Angiomotorik stimuliert und damit den Abfluss über das Lymphsystem anregt.

Vorzugsweise wird mit kontinuierlichen oder randomisierten Frequenzbändern gearbeitet, wodurch die Stimulation verschiedenartiger Gewebe- und Nervenstrukturen erreicht wird.

Durch einen günstigerweise vorgesehenen Fußschalter kann ein Umschalten der Arbeitsparameter, z.B. der Frequenzen, auch dann bewerkstelligt werden, wenn der Therapeut mit beiden Händen, also z.B. mit zwei Handschuhelektroden, arbeitet oder wenn eine Selbstbehandlung stattfindet.

Die erfindungsgemäße Vorrichtung umfasst auch eine Elektrodenanordnung, an der einerseits die Elektrode mit dem relativ hohen Übergangswiderstand realisiert ist, die über die Haut des Patienten geführt wird, und andererseits die Metallelektrode mit dem geringen Übergangswiderstand, welche entweder mechanisch abdeckbar oder elektrisch abschaltbar ist, so dass sie selektiv nur dann wirksam wird, wenn eine Selbstbehandlung stattfindet und die Metallelektrode beim Halten und Bewegen von der Hand der jeweiligen Person berührt wird.

Die Elektrodeanordnung kann einen Handgriff umfassen, in den die eigentliche hochohmige Elektrode lösbar einsetzbar ist.

Vorteilhafterweise kann insoweit ein Federkorbstecker an der hochohmigen Elektrode angebracht sein, der in eine korrespondierende Hülse am Griff eingesteckt werden kann.

Die Elektrodenplatte der hochohmigen Elektrode ist mit einem elektrisch leitfähigen Schaumstoff bedeckt, welche mit einer Kunststoff-Folie, vorzugsweise auf Vinylbasis abgedeckt ist, wobei erfindungsgemäß zwischen Schaumstoff und Kunststoff-Folie eine Hydrogel-Scheibe eingesetzt ist, welche sicherstellt, dass der angestrebte Effekt besonders gut realisiert wird.

Nachfolgend wird die Erfindung anhand eines bevorzugten Ausführungsbeispiels in Verbindung mit der Zeichnung näher erläutert. Dabei zeigen:
- Fig. 1: ein Blockschaltbild einer erfindungsgemäßen Vorrichtung zur Elektrotherapie und
- Fig. 2: einen Längsschnitt durch eine erfindungsgemäße Elektrodenanordnung.

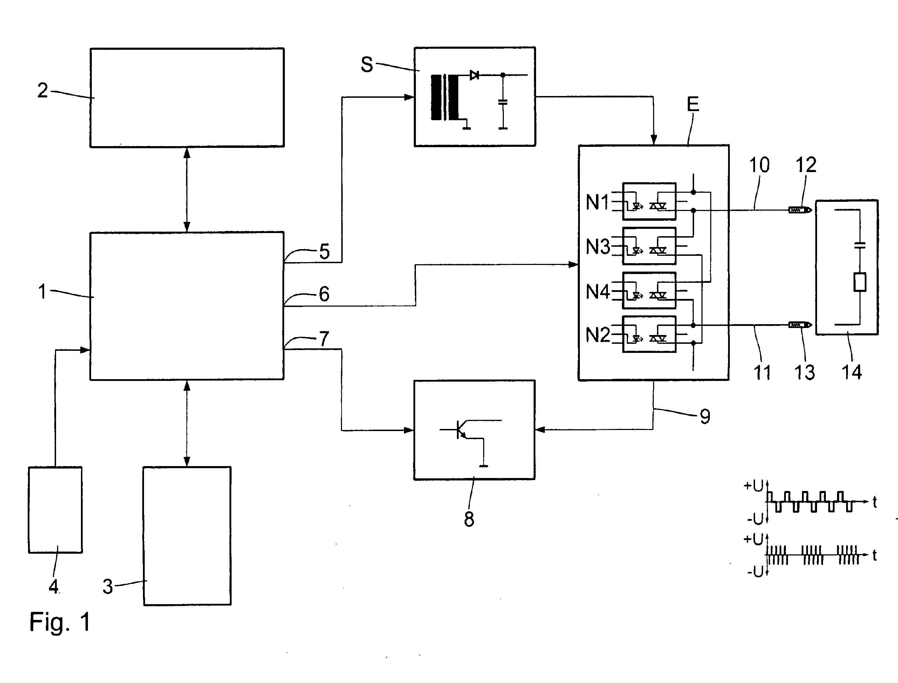

Die in Fig. 1 dargestellte Schaltungsanordnung umfasst ein Mikroprozessorsystem 1, welches verbunden ist mit einer Anzeige- und Bedieneinheit 2, einer Spannungsversorgung 3 und einem Fußschalter 4.

Ein erster Ausgang 5 des Mikroprozessorsystems 1 ist über einen Spannungswandler S mit einer erfindungsgemäßem Umpol- und Entladeschaltung E verbunden, ein zweiter Ausgang 6 ist direkt mit der Schaltung E verbunden und ein dritter Ausgang 7 ist mit einer steuerbaren Konstantstromquelle 8 verbunden, an der auch ein Ausgang 9 der Umpol- und Entladeschaltung E anliegt.

Die Ausgänge 10 und 11 sind über Kontakte 12 und 13 mit einem Patienten bzw. im Blockschaltbild mit einer Patienten-Ersatzschaltung 14 verbunden.

In Fig. 1 rechts unten sind die Impulse an den Ausgängen 10 und 11 veranschaulicht, deren Phase und Frequenz veränderbar ist, und zwar oben die Grund-Impulse und darunter die sogenannten Burst-Impulse.

Die Umpol- und Entladeschaltung E umfasst vier Schaltungsteile N1 bis N4, welche jeweils einen durch einen Optokoppler angesteuerten Thyristor aufweisen.

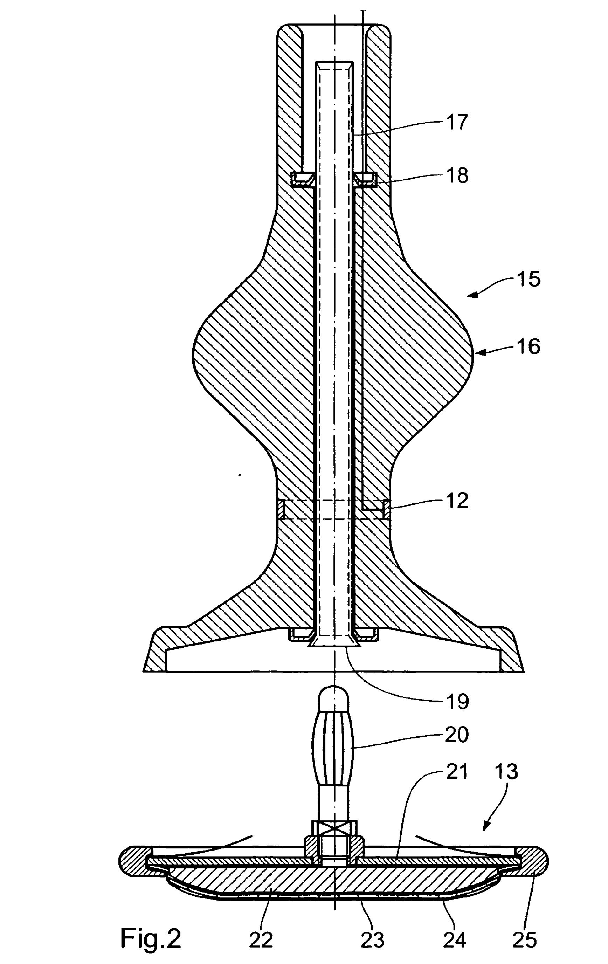

In Fig. 2 ist eine erfindungsgemäße Elektrode 15 dargestellt. Diese umfasst einen ergonomisch geformten Silikonhandgriff 16, in welchem ein Kontaktrohr 17 angeordnet ist, welches über eine Wellensicherung 18 fixiert ist. Das Kontaktrohr 17 ist - in der Zeichnung nicht sichtbar - mit den Ausgängen bzw. Kontakten 12, 13 der Schaltungsanordnung nach Fig. 1 verbindbar.

In das untere offene Ende 19 des Kontaktrohrs 17 ist ein Federkorbstecker 20 einsteckbar, der seinerseits mit einer metallischen Elektrodenplatte 21 verbunden ist, so dass die Elektrodenplatte 21 auf diese Weise am unteren Ende des Silikonhandgriffs 16 festlegbar ist.

Unterhalb der Elektrodenplatte 21 ist eine elektrisch leitfähige Schaumstoffschicht 22 angeordnet und unterhalb dieser eine Hydrogelscheibe 23, wobei über die gesamte Anordnung eine Membrane 24 aus Vinylfolie gespannt ist, die durch einen Klemmring 25 festgelegt ist.

## Patentansprüche

1. Vorrichtung zur Elektrotherapie mit einer Schaltungsanordnung mit einer Endstufe zur Erzeugung einer Folge von Spannungsimpulsen und mit zwei Elektroden zum Anlegen dieser Spannungsimpulse an ein zu behandelndes Körperteil, wobei eine der beiden Elektroden mit einer Oberfläche mit einem relativ hohen Übergangswiderstand und die andere Elektrode mit einem relativ geringen Übergangswiderstand zur menschlichen Haut ausgebildet ist, wobei die Elektrode mit der Oberfläche mit dem hohen Übergangswiderstand über das zu behandelnde Körperteil bewegbar ist und die Oberfläche durch eine Kunststoffschicht gebildet ist, wobei Impulsfolgen mit Spannungen von 1 bis 600 V erzeugt werden und im Gewebe ein Strom im Mikroampere-Bereich hervorgerufen wird, **dadurch gekennzeichnet, dass** eine Schnellentladeschaltung (E) vorgesehen ist, die mehrere durch Optokoppler angesteuerte Thyristoren (N bis N4) umfasst, wobei einer Grundfrequenz in der Größenordnung von 100 Hz eine Frequenz in der Größenordnung von 1 bis 10 Hz überlagert ist.

2. Vorrichtung nach Anspruch 1, **dadurch gekennzeichnet, dass** die Endstufe kontinuierliche oder randomisierte Frequenzbänder erzeugt.

3. Vorrichtung nach Anspruch 1, **dadurch gekennzeichnet, dass** ein Fußschalter (4) zum Umschalten der Arbeitsparameter, z.B. der Frequenzen, Amplituden usw. vorgesehen ist.

4. Vorrichtung nach Anspruch 1, **dadurch gekennzeichnet, dass** eine Elektrodenanordnung vorgesehen ist, an der einerseits die Elektrode (15) mit dem relativ hohen Übergangswiderstand und andererseits eine Metallelektrode (21) mit geringem Übergangswiderstand ausgebildet sind, wobei die Elektrode (15) einen Handgriff (16) umfasst, in den die Elektrode (21) mit relativ hohem Übergangswiderstand lösbar einsetzbar ist und wobei die Elektrode (21) mit relativ hohem Übergangswiderstand eine metallische Elektrodenplatte (21), hierauf eine leitfähige Schaumstoffschicht (22) und hierüber eine Kunststoff-Folie (24), insbesondere eine Vinylfolie, umfasst, wobei zwischen Schaumstoffschicht (22) und Kunststoff-Folie (24) eine Hydrogel-Scheibe (23) eingesetzt ist.

5. Vorrichtung nach Anspruch 4, **dadurch gekennzeichnet, dass** die Metallelektrode (21) mechanisch abdeckbar oder elektrisch abschaltbar ist.

6. Vorrichtung nach Anspruch 4, **dadurch gekennzeichnet, dass** die Elektrode (15) einen Handgriff (16) umfasst, in den die Elektrode (21) mit relativ hohem Übergangswiderstand lösbar einsetzbar ist.

7. Vorrichtung nach Anspruch 6, **dadurch gekennzeichnet, dass** ein Federkorbstecker (20) an der Elektrode (21) mit relativ hohem Übergangswiderstand angebracht ist, der in eine korrespondierende Hülse (17) am Griff (16) der Elektrodenanordnung einsetzbar ist.

## Claims

1. Apparatus for electrotherapy, comprising a circuit arrangement with a terminal for generation of a sequence of voltage pulses; and two electrodes for application of these voltage pulses to a part of the body that is to be treated; one of the two electrodes having a surface of comparatively high transition resistance and the other electrode having a comparatively low transition resistance towards human skin; the electrode with the surface of high transition resistance being movable over the treated part of the body and the surface being a plastic film; with pulse sequences of voltages in a range from 1 to 600 V being generated and a current in a microampere range being produced in human tissue; **characterized in that** a rapid-discharge circuit (E) is provided, including several thyristors (N1 to N4) which are triggered by optocouplers, wherein a base frequency of 5 to 250 Hz, preferably approximately 100 Hz, is superimposed by a frequency of 1 to 30 Hz.

2. Apparatus according to claim 1, **characterized in that** the terminal produces continuous or randomized frequency bands.

3. Apparatus according to claim 1, **characterized in that** a foot-actuated switch (4) is provided for switch-over of working parameters, for example frequencies, amplitudes etc.

4. Apparatus according to claim 1, **characterized in that** an electrode arrangement is provided, comprising the electrode (15) of comparatively high transition resistance on the one hand and a metal electrode (21) of low transition resistance on the other, wherein the electrode (15) comprises a handle (16) into which the electrode (21) with comparatively high transition resistance is releasably insertable, and wherein the electrode (21) of comparatively high transition resistance comprises a metal electrode plate (21) with a conductive foam layer (22) provided thereon that is covered by a plastic film (24), in particular a vinyl film, a hydrogel disk (23) being inserted between the foam layer (22) and the plastic film (24).

5. Apparatus according to claim 4, **characterized in that** the metal electrode (21) is covered mechanically or switched off electrically.

6. Apparatus according to claim 4, **characterized in that** the electrode (15) comprises a handle (16) into which the electrode (21) of comparatively high transition resistance is releasably insertable

7. Apparatus according to claim 6, **characterized in that** a spring-basket-type plug (20) is mounted on the electrode (21) of comparatively high transition resistance, the spring-basket-type plug (20) being insertable into a corresponding sleeve (17) on the handle (16) of the electrode arrangement.

## Revendications

1. Dispositif d'électrothérapie comportant une disposition de circuit ayant un étage de sortie pour générer une séquence d'impulsions de tension et deux électrodes pour appliquer cette impulsion de tension à une partie corporelle à traiter, l'une des deux électrodes étant constituée avec une surface présentant une résistance de transition relativement élevée, et l'autre électrode présentant une résistance de transition relativement élevée par rapport à la peau humaine, l'électrode avec la surface présentant la résistance de transition élevée pouvant être déplacée sur la partie corporelle à traiter et la surface étant formée par une couche de matière plastique, les séquences d'impulsion étant générées avec des tensions de 1 à 600 V et entraînant dans les tissus un courant dans un domaine de micro-ampères, **caractérisé en ce qu'**un circuit de décharge rapide (E) est prévu, qui comprend plusieurs thyristors (N1 à N4) commandés par optocoupleur (N1 à N4), une différence fondamentale dans l'ordre de grandeur de 100 Hz étant superposée à une fréquence d'un ordre de grandeur de 1 à 10 Hz.

2. Dispositif selon la revendication 1, **caractérisé en ce que** l'étage de sortie génère des bandes de fréquence continues ou randomisées.

3. Dispositif selon la revendication 1, **caractérisé en ce qu'**un commutateur au pied (4) est prévu pour commuter les paramètres de travail, par exemple les fréquences, les amplitudes, etc.

4. Dispositif selon la revendication 1, **caractérisé en ce qu'**une disposition d'électrodes est prévue, sur laquelle d'une part les électrodes (15) sont formées avec la résistance de transition relativement élevée et d'autre part, une électrode métallique (21) avec une résistance de transition faible, l'électrode (15) comprenant une poignée (16) dans laquelle l'électrode (21) est utilisable avec une résistance de transition relativement élevée amovible et l'électrode (21) avec une résistance de transition élevée comprenant une plaque d'électrodes métallique (21), d'un côté une couche de mousse conductrice (22) et de l'autre côté, une feuille de plastique (24), en particulier une feuille de vinyle, un disque d'hydrogel (23) étant placé entre la couche de mousse (22) et la feuille de plastique (24).

5. Dispositif selon la revendication 4, **caractérisé en ce que** l'électrode métallique (21) peut être recouverte mécaniquement ou peut être commutée électriquement.

6. Dispositif selon la revendication 4, **caractérisé en ce que** l'électrode (15) comprend une poignée (16) dans laquelle l'électrode (21) est utilisable avec une résistance de transition relativement élevée amovible.

7. Dispositif selon la revendication 6, **caractérisé en ce qu'**une fiche à ressort (20) est connectée à l'électrode (21) avec une résistance de transition relativement élevée qui peut être utilisée dans une gaine correspondante (17) sur la poignée (16) de la disposition d'électrodes.
